# EUROPÄISCHE PATENTSCHRIFT

(11) **EP 0 497 157 B1**
(45) Veröffentlichungstag und Bekanntmachung des Hinweises auf die Patenterteilung: **16.10.1996**
(21) Anmeldenummer: 92100700.1
(22) Anmeldetag: 17.01.1992
(51) Int. Cl.: A61K 6/083, A61C 13/087

(54) **Vernetzte Dentalformkörper**
Crosslinked molded dental articles
Objets dentaires réticulés

(30) Priorität: 30.01.1991 DE 4102627
(43) Veröffentlichungstag der Anmeldung: 05.08.1992
(73) Patentinhaber: Heraeus Kulzer GmbH & Co.KG, 63450 Hanau (DE)
(72) Erfinder: Müller, Michael, Dr., W-5060 Bergisch Gladbach 2 (DE); Podszun, Wolfgang, Dr., W-5000 Köln 80 (DE); Bebermeier, Günther, W-5090 Leverkusen 3 (DE); Richter, Roland, Dr., W-5090 Leverkusen (DE)
(74) Vertreter: Kühn, Hans-Christian

(56) Entgegenhaltungen:
- EP-A- 0 127 758
- EP-A- 0 438 629
- GB-A- 2 189 793
- US-A- 4 396 377

## Beschreibung

Die Erfindung betrifft ein Verfahren zur Herstellung von vernetzten Dentalformkörpem, insbesondere von Kunststoffzähnen, die hierzu verwendeten härtbaren Zusammensetzungen sowie die erhaltenen Gegenstände selbst.

Unvernetzte Kunststoffzähne lassen sich durch thermoplastische Formgebung aus beispielsweise Polymethylmethacrylat herstellen. Die erhaltenen Zähne erreichen jedoch die geforderten Gebrauchseigenschaften nicht im vollen Umfang, so ist ihre Verschleißfestigkeit und Krakeleebeständigkeit unzureichend.

Durch Photopolymerisation härtbare Zusammensetzungen zur Herstellung von Dentalkörpern sind bereits aus EPA 0 438 629 bekannt geworden. Dieser Stand der Technik lehrt jedoch nicht die erfindungsgemäße Verarbeitung der Kompositionen zu Formkörpern, die in einer zweiten Verfahrensstufe dreidimensional zu Duroplasten vernetzt werden. Aus der GBA 2 189 793 sind plastische Massen bekannt geworden, aus denen zwar dentale Restaurationsmaterialien hergestellt werden können, die sich aber nicht zur thermoplastischen Verarbeitung gemäß der vorliegenden Erfindung eignen.

Übliche Kunststoffzähne werden durch Polymerisation von Mischungen aus
- 35-50 Gew.-%: Monomerflüssigkeit, enthaltend 80-95 Gew.-% Methylmethacrylat und 5-20 Gew.-% Ethylenglykoldimethacrylat als Vernetzer
und
- 50-65 Gew.-%: Pulver aus unvernetztem Polymethylmethacrylat in Form von Perlpolymerisaten mit einer mittleren Teilchengröße von 30-120 µm
hergestellt.

In der US 4 396 377 werden Dentalmaterialien zur Herstellung von Kunststoffzähnen vorgeschlagen, die als Pulver (neben unvernetztem) vernetztes Polymethylmethacrylat enthalten. Als Monomerflüssigkeit wird das herkömmliche Gemisch aus Methylmethacrylat und einem Vernetzer wie Ethylenglykoldimethacrylat verwendet. Dentalmaterialien gemäß US 4 396 377 weisen die folgende Zusammensetzung auf:
0-50 % unvernetztes Polymer
10-70% vernetztes Polymer
20-66 % Monomer (welches nicht als Vernetzer wirkt)
7-27 % Vernetzer

Die EP-A 59 525 beschreibt ähnliche Materialien wie die vorstehend zitierte Patentschrift. Die beanspruchten Materialien haben die folgende Zusammensetzung:
0-50 % unvernetztes Polymer
10-70 % vernetztes Polymer
2-30 % Monomer (welches nicht als Vernetzer wirkt)
20-70 % Vernetzer

Aus der DE-A 3 820 497 und EP-A 110 092 sind Dentalwerkstoffe zur Herstellung von künstlichen Zähnen bekannt geworden, bei denen als Monomerflüssigkeit ausschließlich oder überwiegend Vernetzer und als Polymerkomponente ausschließlich vernetztes Polymerisat mit besonderen Quellungseigenschaften Verwendung findet. Diese Dentalwerkstoffe haben die nachfolgende Zusammensetzung:
5-35 % vernetztes Polymer
0-40 % Monomer (welches nicht als Vernetzer wirkt)
40-90 % Vernetzer.

Die DE-A 2 403 211 beschreibt füllstotffhaltige Dentalmassen, die dadurch gekennzeichnet sind, daß sie als Füllstoff ausschließlich mikrofeines Siliziumdioxid und als Monomer BisGMA oder spezielle durch Umsetzung von Diisocyanaten mit Hydroxyalkylmethacrylaten erhaltene Urethanmethacrylate enthalten.

Vernetzte Kunststoffzähne werden nach dem sogenannten chemoplastischen Verfahren hergestellt. Bei diesem Verfahren werden Mischungen aus pulverförmigem Polymerisat, Monomer und Vernetzer in Metallformen unter Druck bei erhöhter Temperatur polymerisiert. Dabei kann der Zahn entsprechend dem natürlichen Vorbild mehrschichtig aufgebaut werden. Häufig wird ein dreischichtiger Aufbau mit einer Schmelz-, Dentin- und Halsschicht gewählt, bei dem sich die einzelnen Schichten durch die Pigmentierung und den Vernetzergehalt unterscheiden. Zähne, die nach dem chemoplastischen Verfahren erhalten werden, übertreffen die thermoplastisch hergestellten zwar deutlich hinsichtlich Verschleißfestigkeit, doch erreichen sie noch nicht das für Seitenzähne geforderte Niveau (s. Ullmann's encyclopedia of industrial chemistry, Vol. A8 S. 280, Weinheim, New York 1987).

Die beim chemoplastischen Verfahren verwendete Mischung aus Polymerisat-Pulver, Monomer und Vernetzer ist inhomogen, da die Polymerisat-Partikel, die üblicherweise kugelförmig sind und eine mittlere Teilchengröße von 40 bis 100 µm aufweisen, nicht vollständig aufgelöst werden. Infolgedessen ist auch der ausgehärtete Dentalformkörper nicht vollständig homogen, sondern enthält Bereiche aus unvernetztem Polymethylmethacrylat. Diese unvernetzten Bereiche sind mitverantwortlich für die unzureichende Verschleißfestigkeit und Lösungsmittelbeständigkeit.

Das chemoplastische Verfahren hat darüber hinaus weitere Nachteile: die für das Füllen, Aufheizen, Polymerisieren, Abkühlen und Entformen notwendigen Zeiten sind lang; unter industriellen Fertigungsbedingungen betragen die Taktzeiten ca. 20 bis 50 Minuten. Aufgrund dieser langen Taktzeiten ist eine große Anzahl von Einzelformen, verbunden mit hohen Investitionskosten, notwendig. Außerdem ist der Energieeinsatz für das Aufheizen und Abkühlen der Formen hoch.

Da die Zähne nach der Entformung Grate aufweisen, sind aufwendige nachgeschaltete Arbeitsschritte, wie z.B. Trommeln und Polieren notwendig. Insgesamt gesehen ist das chemoplastische Verfahren weit weniger wirtschaftlich als das thermoplastische.

Das bekannte chemoplastische Verfahren ist hinsichtlich der Variationsmöglichkeiten der eingesetzten Ausgangsmaterialien stark eingeschränkt. So können als Vernetzer nur einfache Alkylendimethacrylate, die das Polymerisat-Pulver bei niedriger Temperatur anlösen, verwendet werden.

Die Aufgabe der Erfindung war die Bereitstellung eines Verfahrens zur Herstellung vernetzter Dentalformkörper, das die vorstehend beschriebenen Nachteile nicht aufweist. Diese Aufgabe wird durch ein spezielles Verfahren, das auf einer thermoplastischen Formgebung und anschließender Vernetzung durch Photopolymerisation beruht, gelöst.

Gegenstände der Erfindung sind somit vernetzte Dentalformkörper sowie ein Verfahren zur Herstellung dieser Dentalformkörper, das dadurch gekennzeichnet ist, daß eine Mischung enthaltend
- a) 40 bis 90 Gew.-%: Polymerkomponente mit einem Löslichkeitsparameter von 8 bis 12,5 (cal/cm³)^{1/2} aus 40 bis 100 Gew.-% unvernetztem und 0 bis 60 Gew.-% vernetztem Polymer
- b) 10 bis 60 Gew.-%: Monomerkomponente enthaltend 0 bis 90 Gew.-% monofunktionelle (Meth)acrylsäureester und 10 bis 100 Gew.-% polyfunktionelle (Meth)acrylsäureester
und
- c) 0,1 bis 5 Gew.-%: Photoaktivator
thermoplastisch zu Formkörpern verarbeitet und anschließend durch Photopolymerisation vernetzt wird.

Bei den erfindungsgemäßen vernetzten, Dentalformkörpern handelt es sich z.B. um künstliche Zähne, Kronen, Brücken, Verblendschalen, Inlays und Onlays. Besonders vorteilhaft kann das neue Verfahren für die Herstellung von künstlichen Zähnen angewendet werden.

Die erfindungsgemäße eingesetzte Polymerkomponente (a) besitzt einen Löslichkeitsparameter von 8 bis 12,5, vorzugsweise 8,5 bis 12 (cal/cm³)^{1/2}. Löslichkeitsparameter wichtiger bekannter Polymere sowie Berechnungsmethoden für neue Polymerzusammensetzungen sind in der Literatur, beispielsweise im Polymer Handbook 3rd. Edition, Brandrup und Immergut, John Weley and Sons, New York, 1989, beschrieben.

Geeignete Polymere sind zum Beispiel Polyester, Polycarbonate, Polyester-Polycarbonat-Copolymere und Vinylpolymere, wie Styrol-Acrylat-Copolymere, Styrol-Acrynitril-Acrylat-Terpolymere und Polyvinylester. Bevorzugte Polymere sind Homo- und Copolymerisate von (Meth)acrylsäureestern. Besonders bevorzugt ist Polymethylmethacrylat und Copolymerisate von Methylmethacrylat mit C₁-C₈-Alkyl(meth)acrylaten als Comonomer. Gut geeignete Comonomere sind Ethylacrylat, n-Butylacrylat, iso-Butylacrylat, n-Butylmethacrylat, iso-Butylmethacrylat, Ethylhexylacrylat, n-Octylacrylat und Ethylhexylmethacrylat.

Die Monomerkomponente (b) enthält 0 bis 90 Gew.-%, vorzugsweise 0 bis 80 Gew.-%, besonders bevorzugt 0 bis 60 Gew.-%, monofunktionelle (Meth)acrylsäureester und 10 bis 100 Gew.-%, vorzugsweise 20 bis 100 Gew.-%, besonders bevorzugt 40 bis 100 Gew.-%, polyfunktionelle (Meth)acrylsäureester.

Geeignete monofunktionelle Methacrylsäureester sind z.B. Methylmethacrylat, Ethylacrylat, n-Propylmethacrylat, iso-Propylmethacrylat, n-Butylacrylat, n-Butylmethacrylat, iso-Butylmethacrylat, n-Hexylmethacrylat, Ethylhexylacrylat, Ethylhexylmethacrylat, n-Octylmethacrylat, Cyclohexylacrylat, Cyclohexylmethacrylat, Phenylethylacrylat, Phenylethylmethacrylat, 2-Methoxyethylmethacrylat, Triethylenglycolmonomethacrylat, 3-Methoxybutylmethacrylat, Butoxyethylacrylat, Furfurylmethacrylat und Tetrahydrofurfurylacrylat.

Mit der Bezeichnung polyfunktionelle (Meth)acrylsäureester sind Ester der Acrylsäure und Methacrylsäure mit zwei und mehr polymerisierbaren Doppelbindungen gemeint. Bevorzugt seien Ester von 2- bis 8-wertigen Alkoholen mit 2 bis 30 Kohlenstoffatomen genannt. Besonders bevorzugt werden Epoxid(meth)acrylate und Urethan(meth)acrylate.

Beispielsweise seien (Meth)-acrylsäureester der Formel 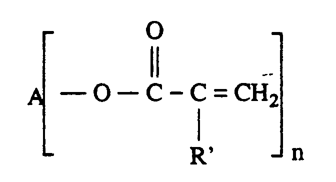 in der
- A: einen geradkettigen, verzweigten, cyclischen, aliphatischen, aromatischen oder gemischt aliphatisch-aromatischen Rest mit 2 bis 30 C-Atomen, der durch -O- oder NH-Brücken unterbrochen und mit Hydroxy, Oxy, Carboxy, Amino oder Halogen substituiert sein kann,
bedeutet,
- R': H oder Methyl bedeutet und
- n: für eine ganze Zahl von 2 bis 8, vorzugsweise 2 bis 4, steht,
genannt.

Vorzugsweise seien Verbindungen der folgenden Formeln genannt: 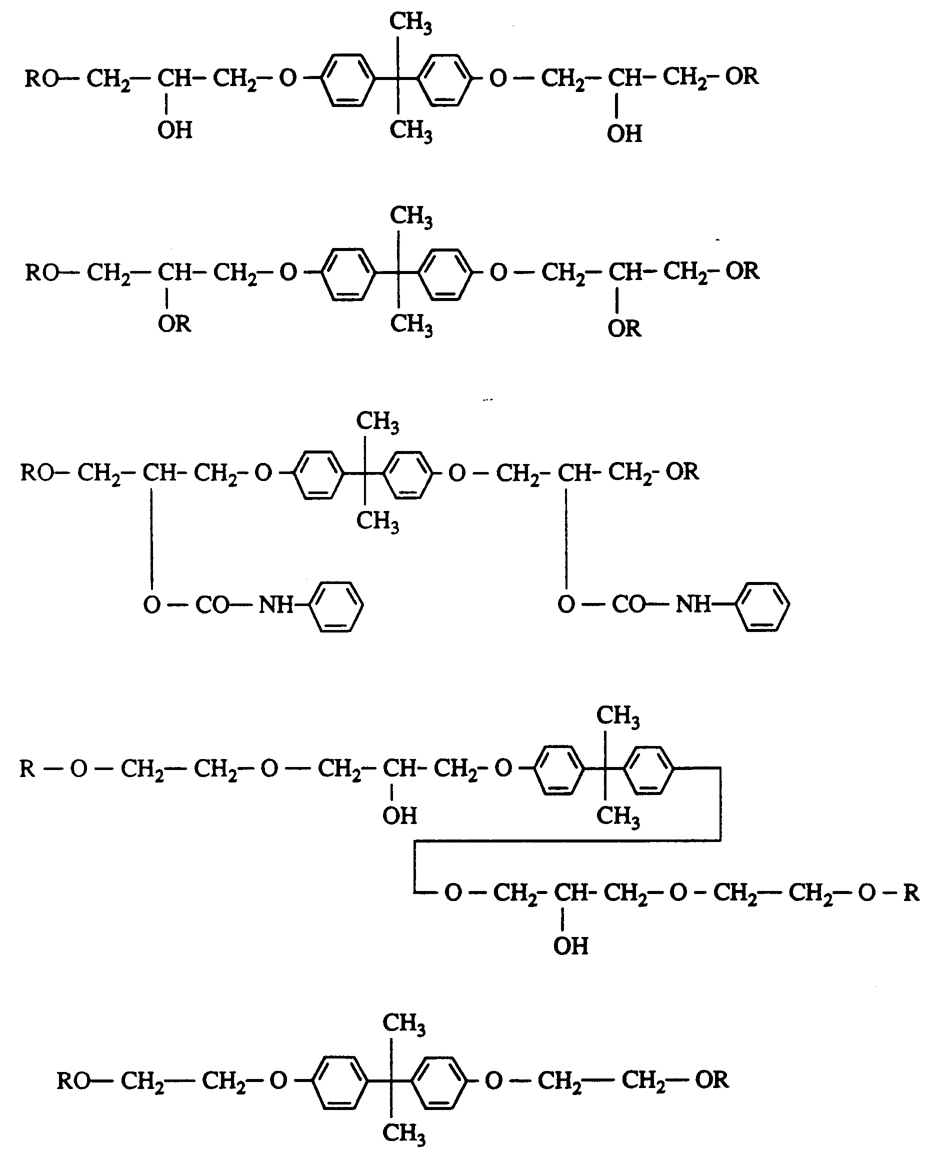 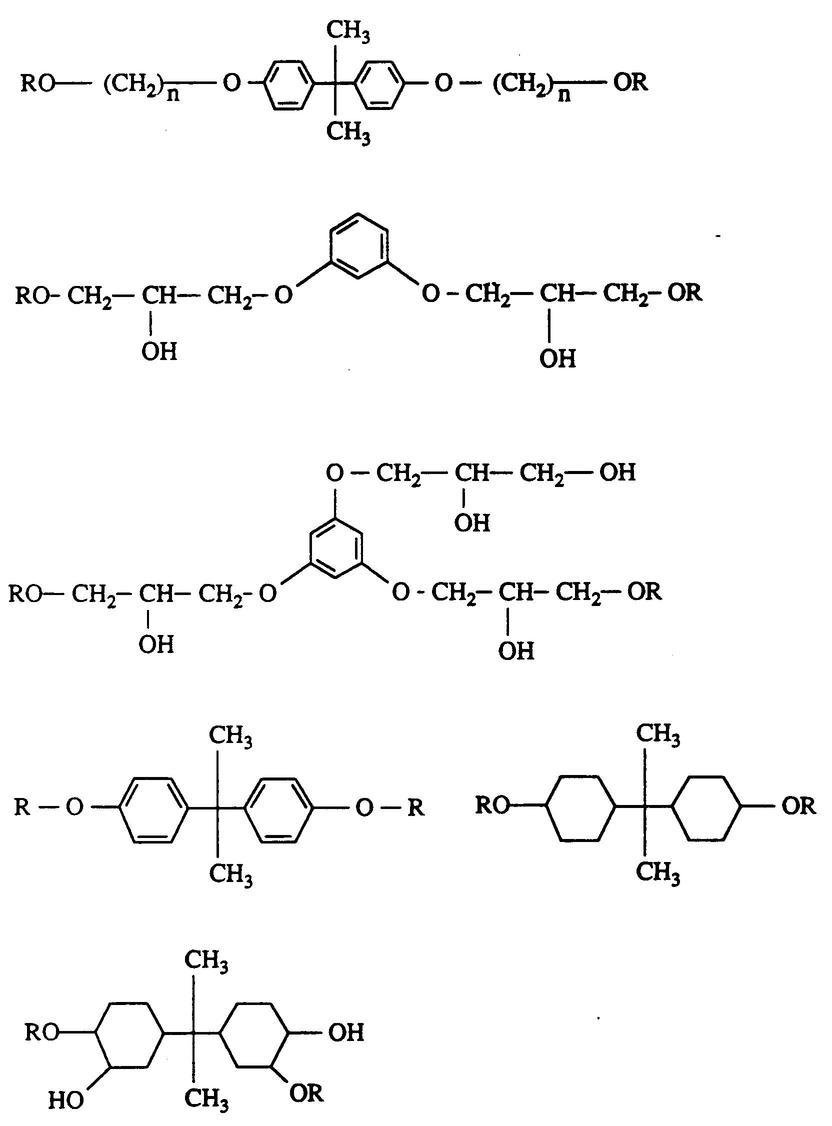 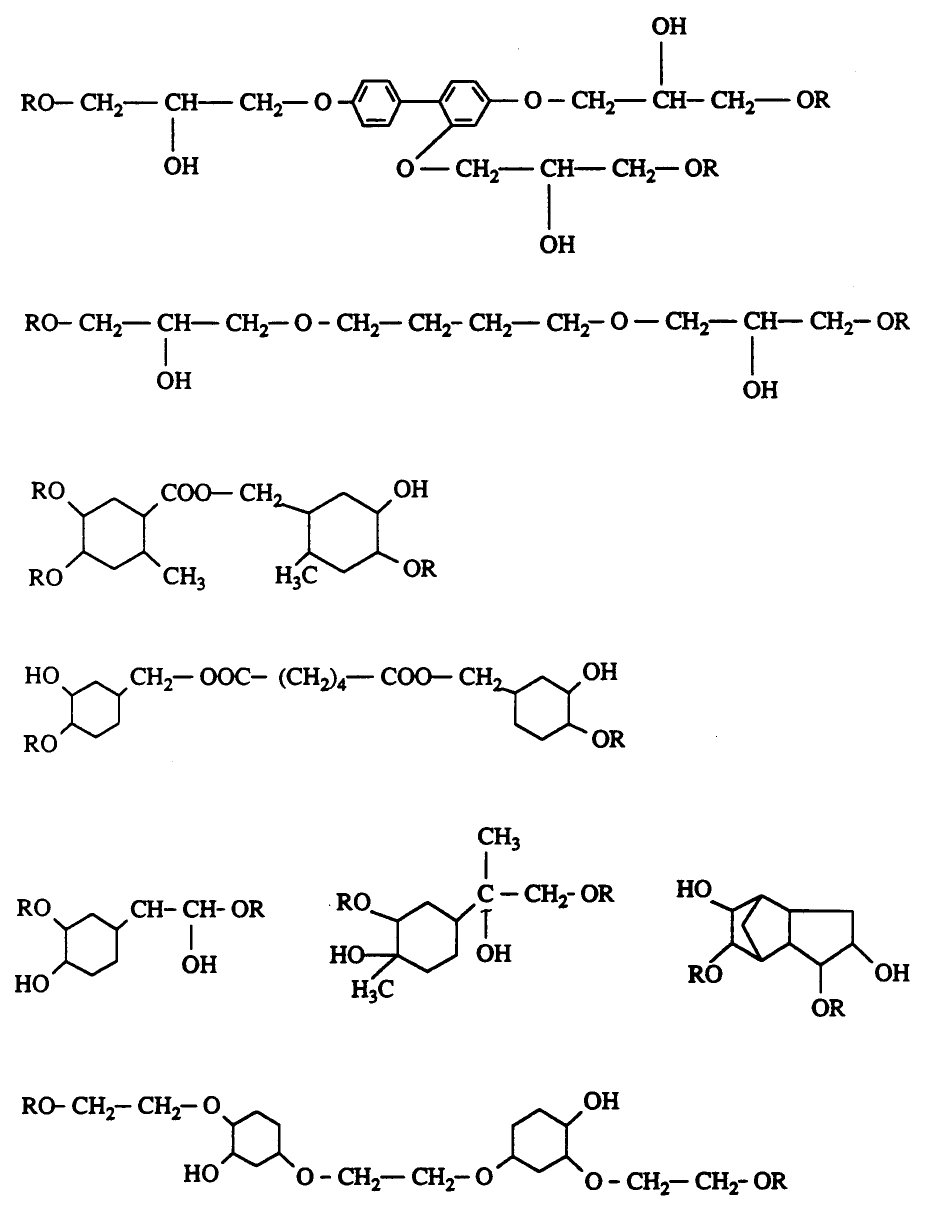 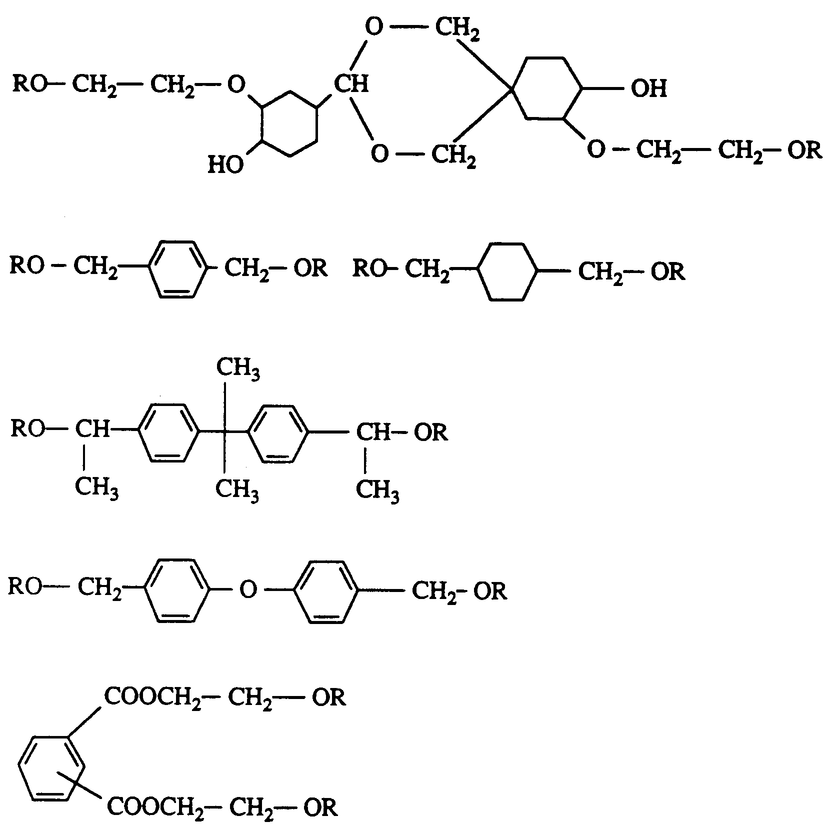 in der ortho-, meta- oder para-Form 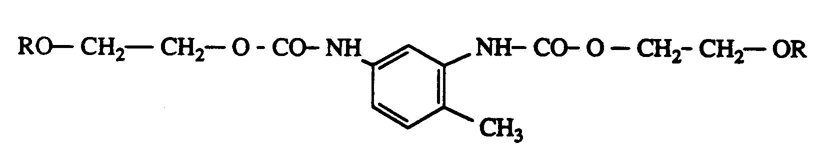 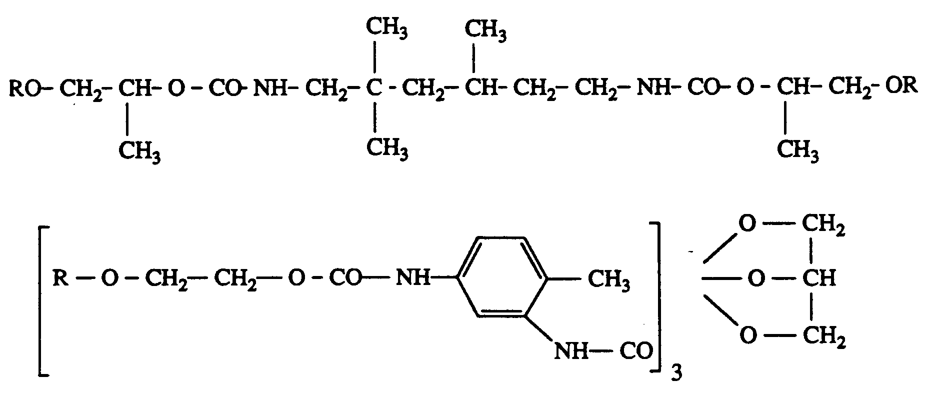 worin R für 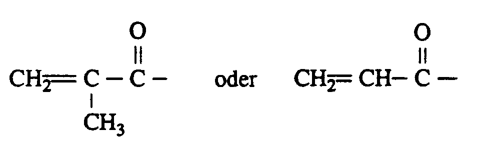 steht.

Außerdem seien Derivate des Tricyclodecans (EP-A 0023686) und Umsetzungsprodukte aus Polyolen, Diisocyanaten und Hydroxyalkylmethacrylaten (DE-A 3703120, DE-A 3703080 und DE-A 3703130) genannt. Beispielsweise seien die folgenden Monomeren genannt:

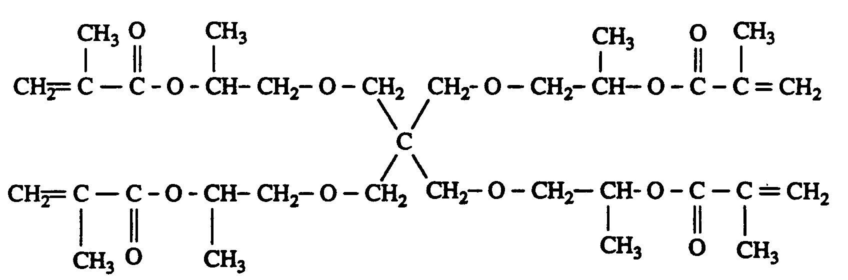

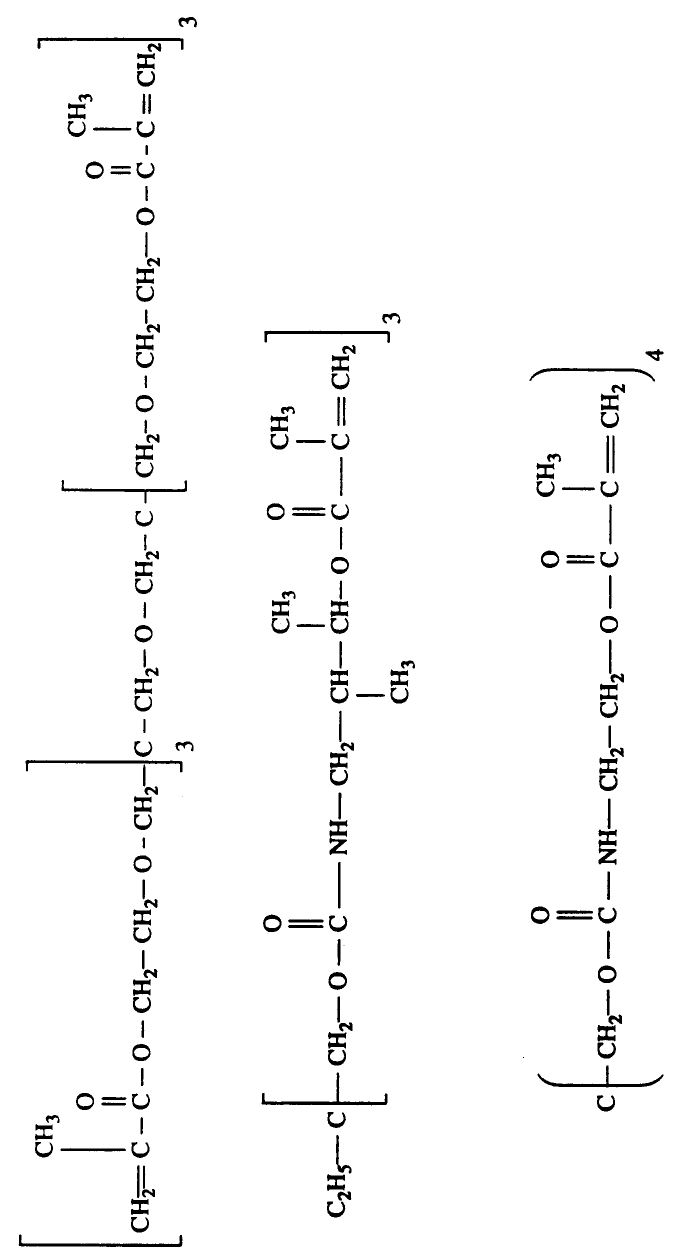

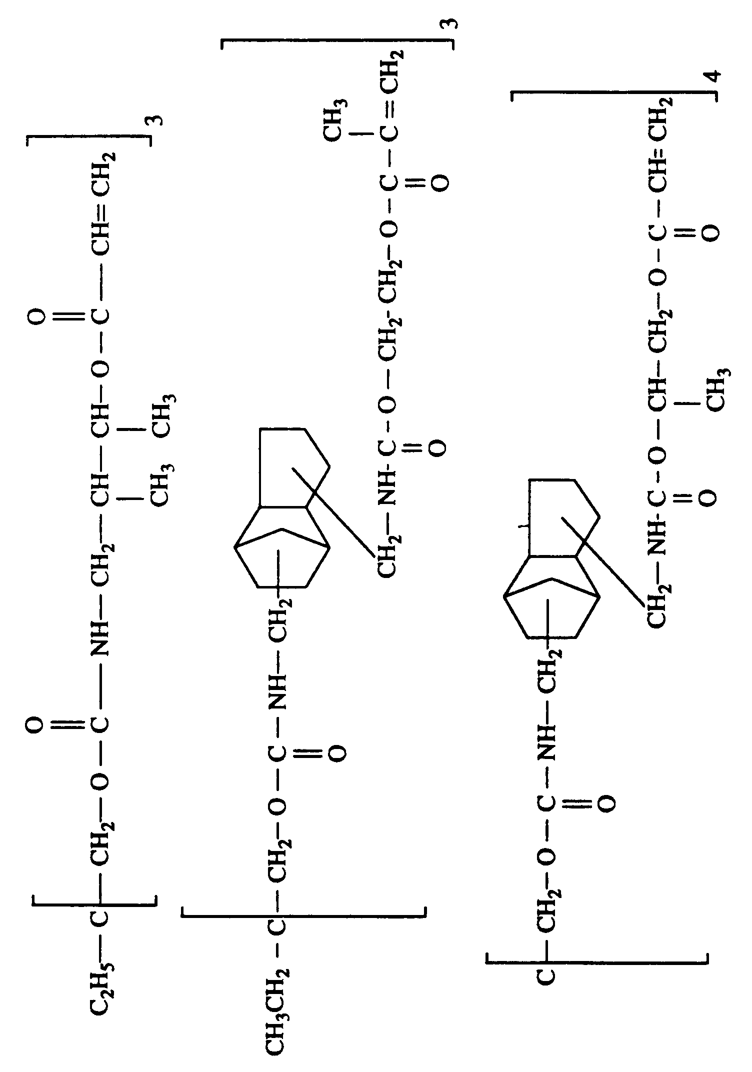

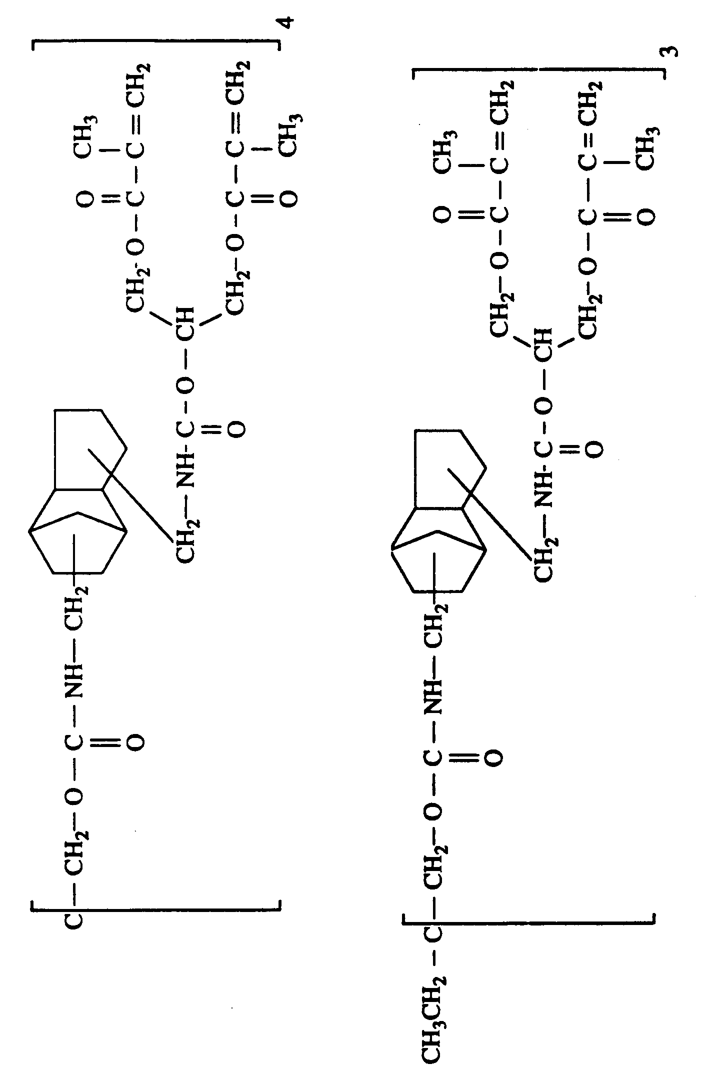

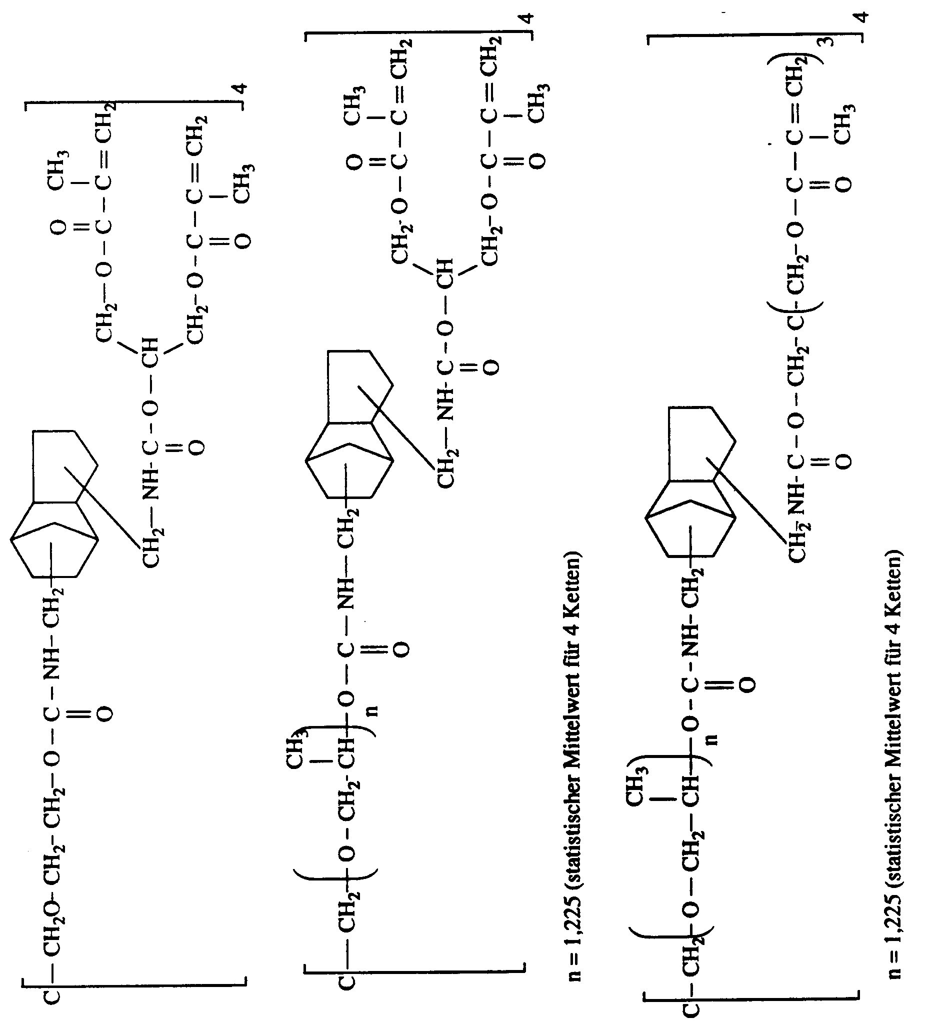

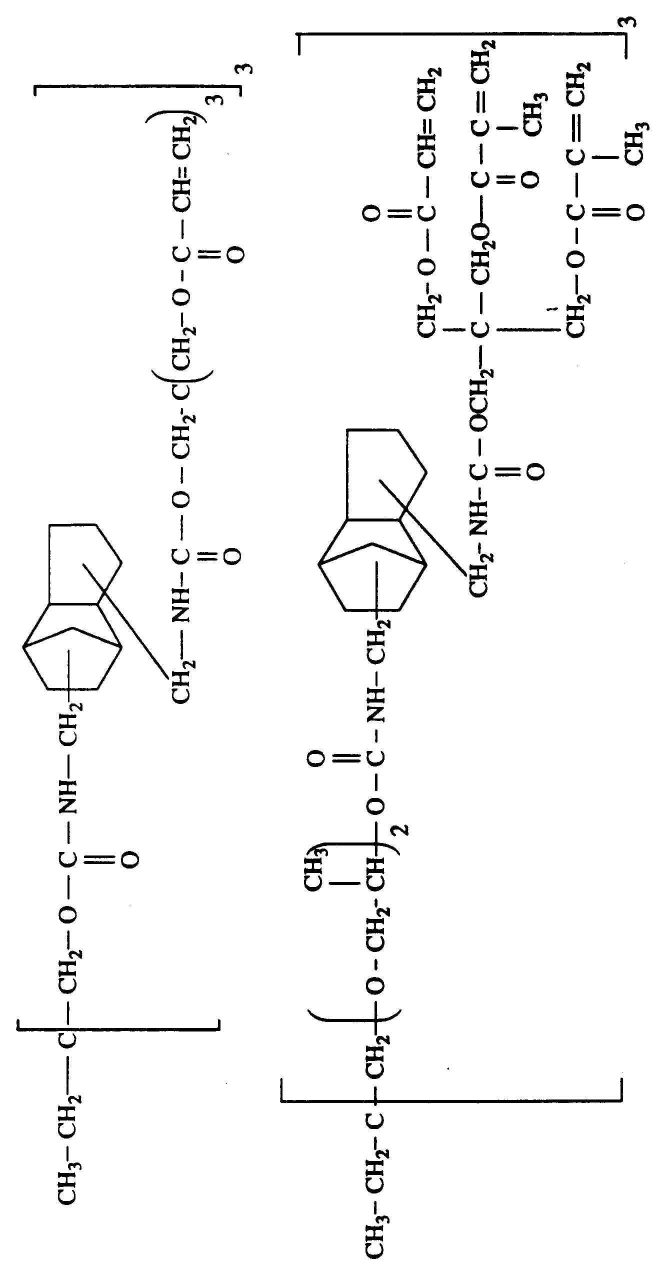

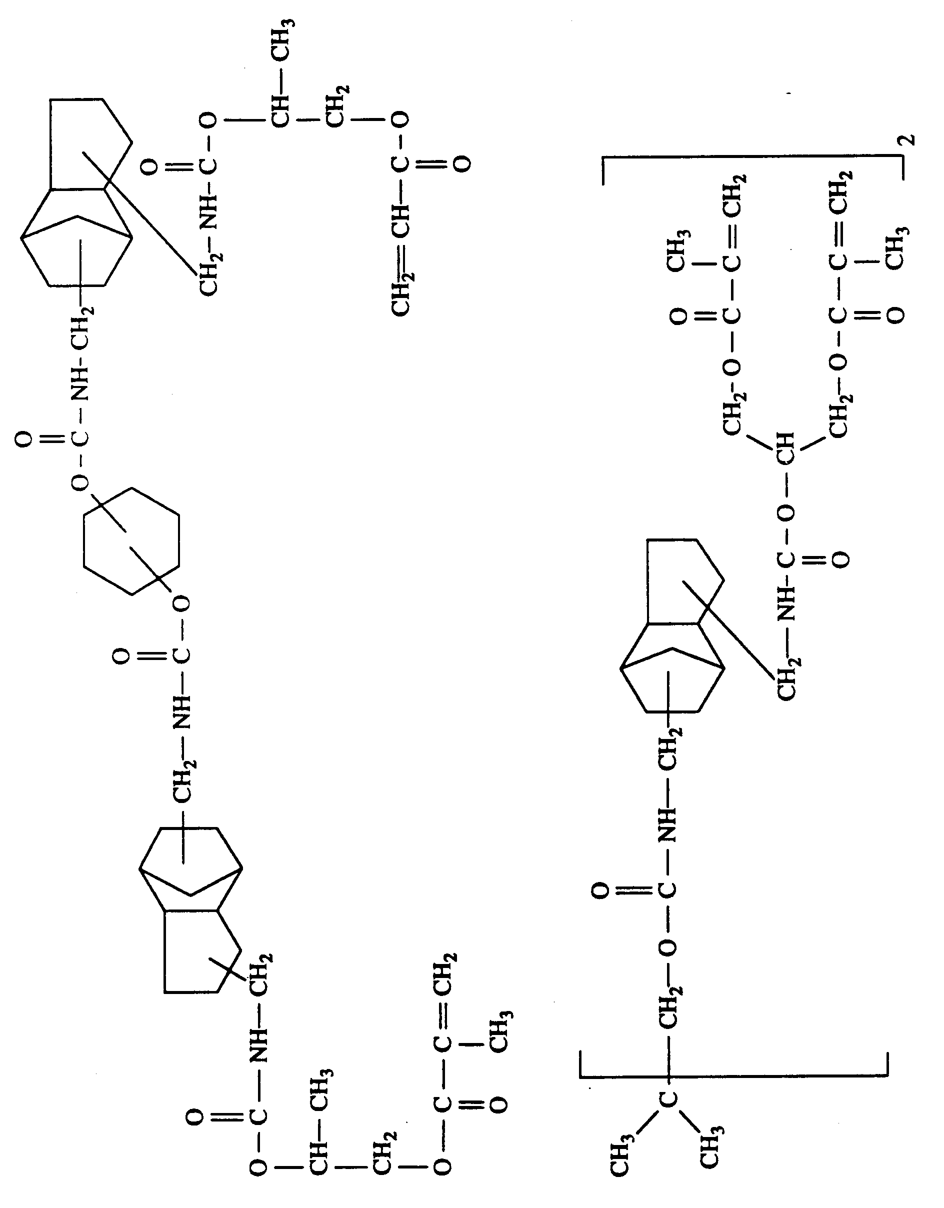

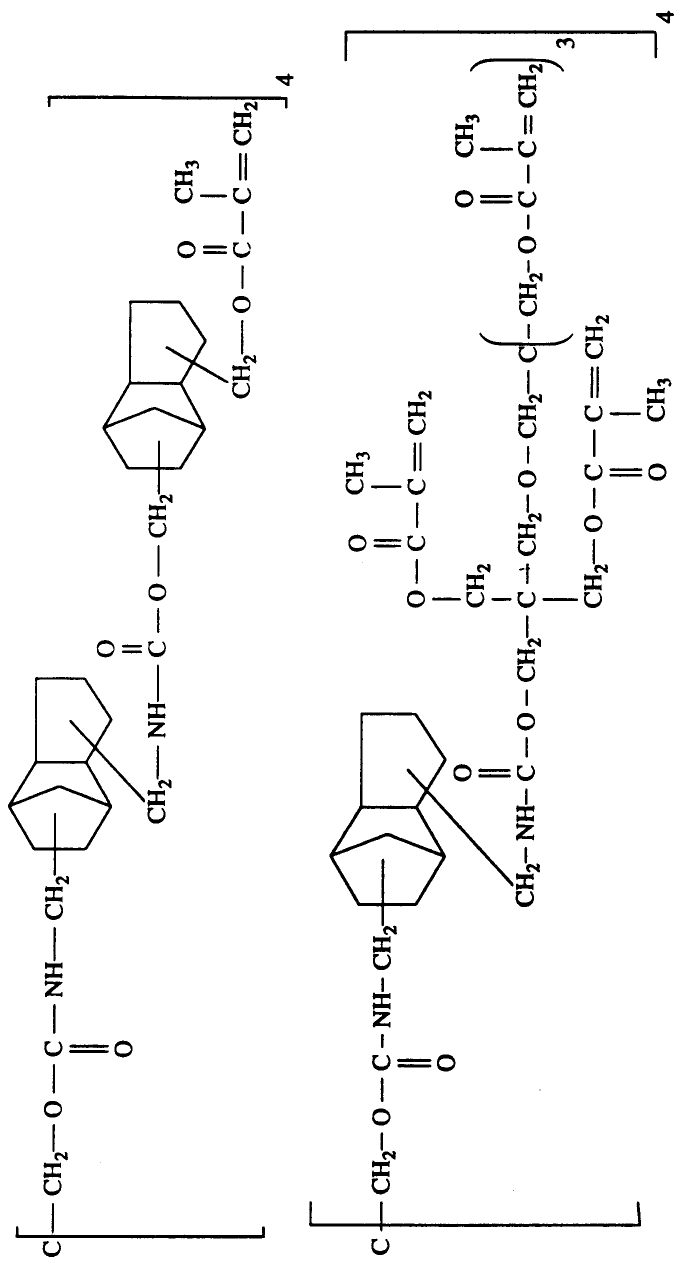

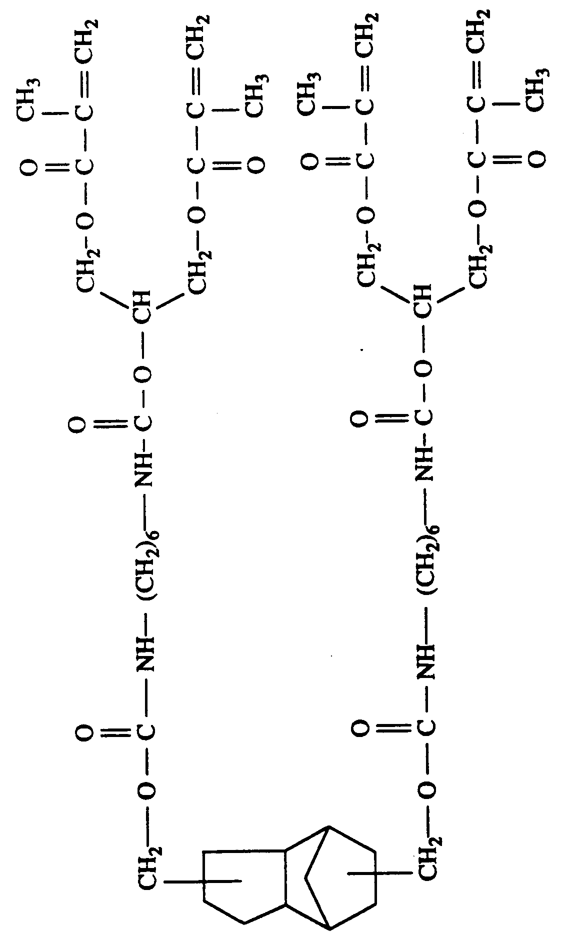

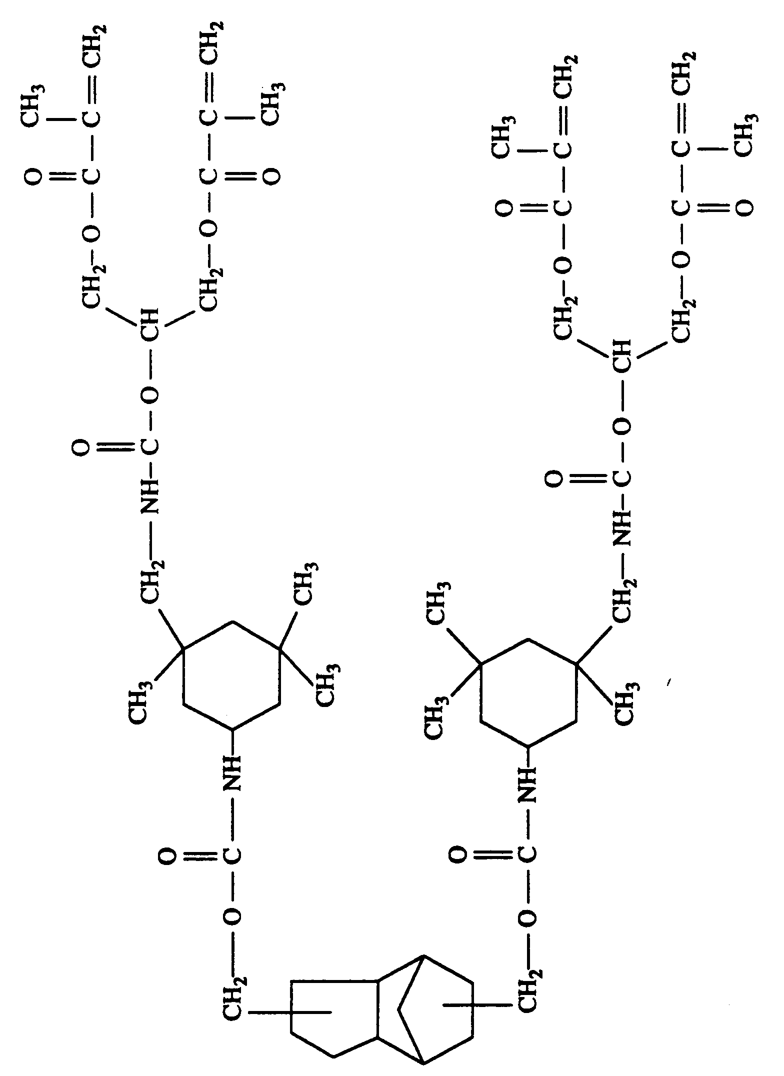

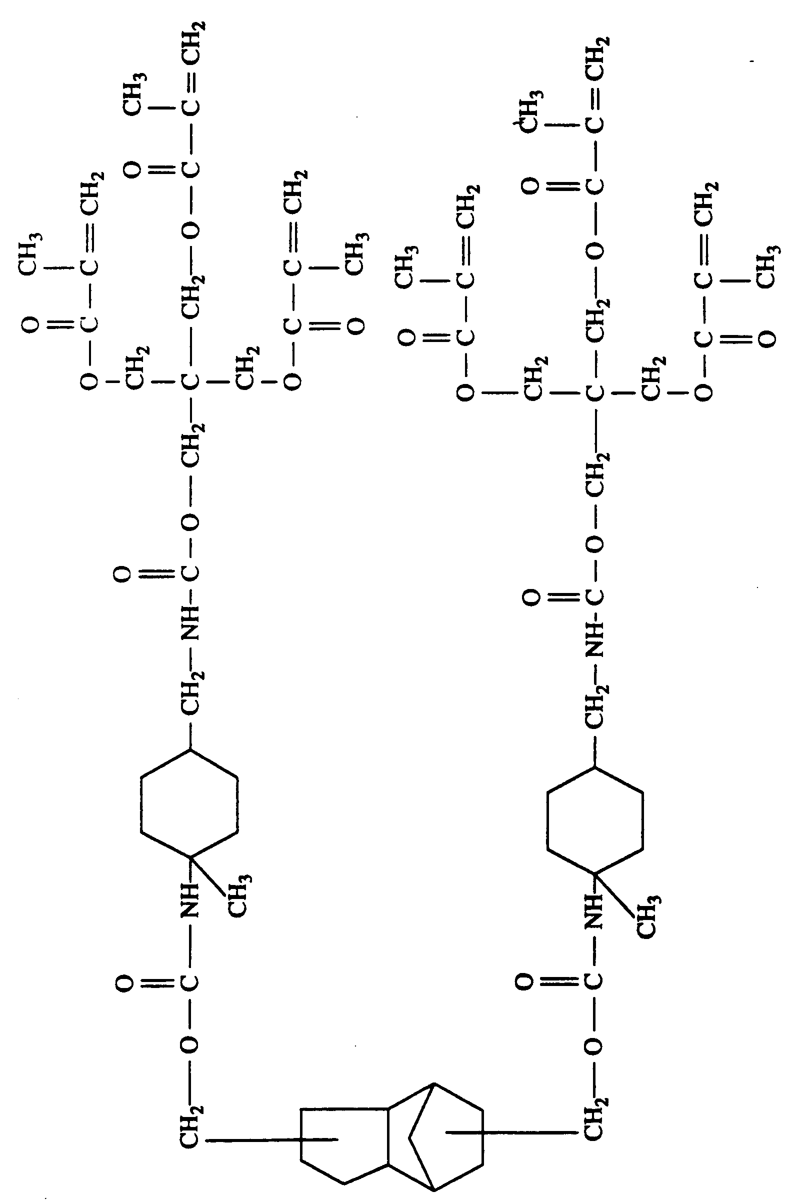

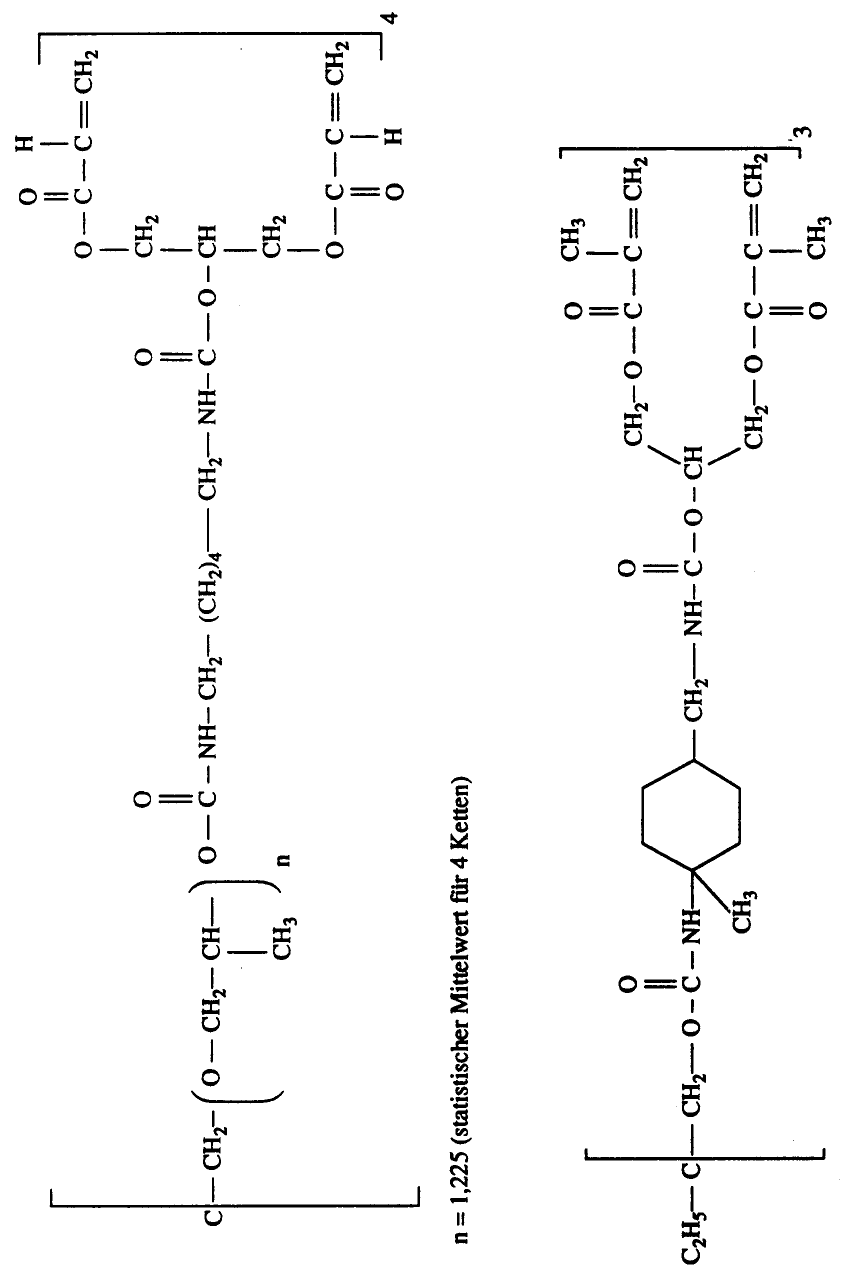

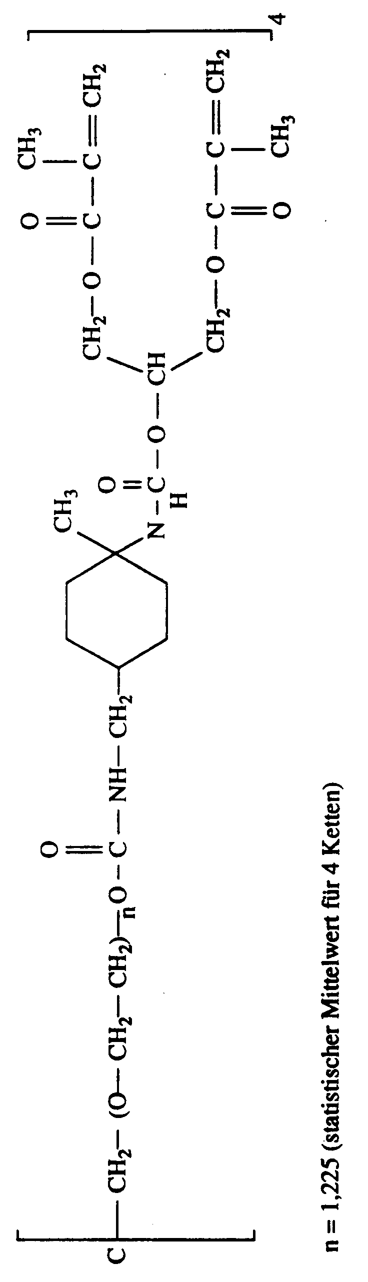

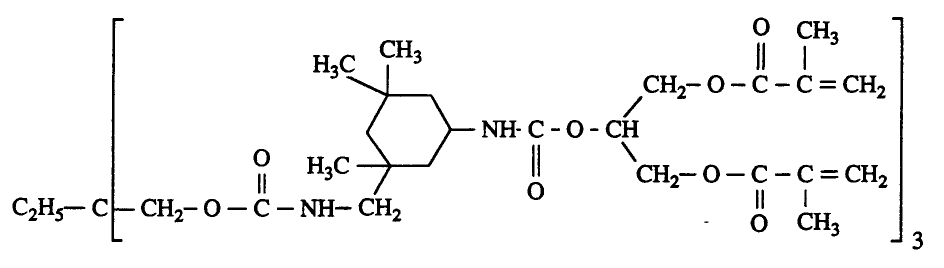

Besonders bevorzugt als (Meth)acrylsäureester wird das sogenannte Bis-GMA der Formel 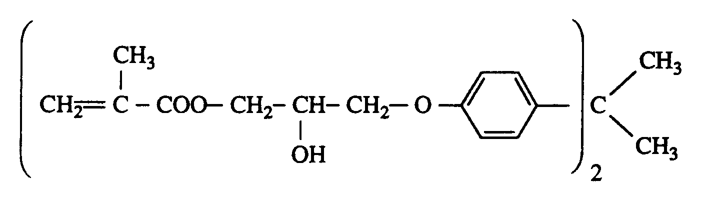 Selbstverständlich ist es möglich, Mischungen verschiedener monofunktioneller und/oder polyfunktioneller (Meth)-acrylsäureester einzusetzen.

Die erfindungsgemäßen Mischungen enthalten im allgemeinen 0,1 bis 5 %, vorzugsweise 0,25 bis 1 %, eines Photoaktivators. Der Photoaktivator besteht aus einem Photopolymerisationsinitiator und gegebenenfalls zusätzlich einem Coaktivator.

Photopolymerisationsinitiatoren im Rahmen der vorliegenden Erfindung sind Radikalbildner, die unter der Einwirkung von Licht, beispielsweise UV-Licht, sichtbarem Licht oder Laserlicht, eine radikalische Polymerisation auslösen.

Photopolymerisationsinitiatoren sind an sich bekannt (s. z.B. Houben Weyl, Methoden der organischen Chemie, Band E 20, S. 80ff, Georg Thieme Verlag Stuttgart, 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketoderivate des Norbornans und substituierter Norbornane, wie z.B. Campherchinon, Metallcarbonyle wie Pentacarbonylmangan oder Chinone wie 9,10-Phenanthrenchinon und Naphthochinon.

Im allgemeinen enthält die Mischung neben dem eigentlichen Photopolymerisationsinitiator noch einen Coaktivator, der die Photopolymerisation beschleunigt. Bekannte Coaktivatoren sind beispielsweise Amine wie p-Toluidin, N,N-Dimethyl-p-toluidin, Trialkylamine wie Trihexylamin, Polyamine wie N,N,N',N'-Tetraalkylalkylendiamine, Barbitursäure und Dialkylbarbitursäuren. Gut geeignet sind auch Dimethylaminobenzolsulfonamide gemäß DOS 3 135 113.

Das Verhältnis von Photopolymerisationsinitiator zu Coaktivator beträgt im allgemeinen 1:1 bis 1:3, in vielen Fällen ist 1:2 anwendbar.

Neben den Komponenten a), b) und c) kann die Mischung natürlich als weitere Komponenten alle die Zusätze enthalten, die für die Herstellung von Dentalformkörpern üblicherweise verwendet werden.

So ist es möglich UV-Stabilisatoren zuzusetzen, um das Nachdunkeln während des Alterns zu vermeiden. Ein besonders geeigneter Stabilisator ist 2-Hydroxy-4-methoxybenzophenon. Als weiteres Beispiel sei 2-(2'-Hydroxy-5-methylphenyl)benzotriazol genannt.

Zur Einstellung einer möglichst naturgetreuen Farbe können an sich bekannte Pigmente und Farbstoffe verwendet werden.

Die Mischung aus den Komponenten a), b) und c) kann durch Compoundieren in leistungsfähigen Mischaggregaten, wie Knetern oder Zweiwellenextrudern herge stellt werden. Die Mischtemperatur liegt dabei im allgemeinen im Bereich von 110 bis 180°C, vorzugsweise 120 bis 160°C. Es wurde gefunden, daß unter diesen Bedingungen keine unerwünschte vorzeitige Vernetzung eintritt.

Die Mischung kann auch durch Zusatz von Lösungsmitteln, wie Aceton, Methylethylketon, Tetrahydrofuran, Dioxan, Ethylacetat, Dichlormethan und Trichlormethan erleichtert werden. Ein bevorzugtes Lösungsmittel ist Methylethylketon. Das Lösungsmittel kann nach dem Mischvorgang beispielsweise mit Hilfe eines Ausdampfextruders entfernt werden. Nach der Extrusion wird zweckmäßigerweise ein Granulierschritt angeschlossen.

Die Formgebung erfolgt durch thermoplastische Verarbeitung, vorzugsweise durch Spritzgießen. Die optimalen Temperaturen des Spritzgießprozesses richten sich nach Art und Zusammensetzung der Komponenten a) und b) und sind durch einfache Vorversuche ermittelbar. Beispielsweise wurden für ein Gemisch aus Polymethylmethacrylat und Bis-GMA folgende Temperaturen als besonders günstig ermittelt:

| | |
|---|---|
| Massetemperatur der Schmelze | 130-140°C |
| Temperatur der Düse | 160-170°C |
| Temperatur der Form | 30- 40°C |

In vielen Fällen liegt die optimale Temperatur der Schmelze im Bereich von 110 bis 180°C.

Zur Verarbeitung eignen sich handelsübliche Spritzgießmaschinen mit beispielsweise Zahnformen als Werkzeug. Durch die Verwendung von Blendern und die entsprechende Gestaltung des Werkzeuges können nacheinander mehrere Schichten eingespritzt werden. Die Verbindung der Schichten untereinander ist gut. Die Taktzeit für einen Spritzvorgang beträgt ca. 10 bis 60 s.

Die erhaltenen Dentalformkörper haben nach der Entformung eine ausreichende Festigkeit und Dimensionsstabilität, so daß sie bei der weiteren Bearbeitung nicht verformt oder beschädigt werden.

Die Vernetzung der Formkörper erfolgt unter Einwirkung von Licht. Prinzipiell ist Licht unterschiedlicher Wellenlänge, beispielsweise sichtbares Licht oder UV-Licht geeignet. Natürlich muß das Emmissionsspektrum der Lichtquelle und die spektrale Empfindlichkeit des verwendeten Photoinitiators aufeinander abgestimmt werden.

Als Strahlungsquelle dienen leistungsfähige Lampen oder besonders vorteilhaft sogenannte Lichtöfen, die eine gute Temperaturkontrolle und die Anwendung von Schutzgas erlauben.

Zur Charakterisierung der Photopolymerisation ist die Photo-DSC (Differential Scanning Calorimetry) gut geeignet. Mit dieser Methode lassen sich die Polymerisationswärmen als Maß für die Polymerisationsumsätze für eine gegebene Rezeptur in Abhängigkeit von der Versuchsbedingungen bestimmen. Der Polymerisationsumsatz nimmt im allgemeinen mit steigender Bestrahlungsintensität, Bestrahlungszeit und Temperatur zu.

Es ist besonders vorteilhaft, die Temperatur während der Photopolymerisation zu steigern, beispielsweise von 50°C auf 90°C. Auf diese Weise wird sowohl gute Maßhaltigkeit als auch ein hoher Polymerisationsumsatz erreicht.

Die notwendige Bestrahlungszeit hängt von der Intensität der Strahlungsquelle und der Schichtdicke des Dentalformkörpers ab. Übliche Bestrahlungszeiten liegen im Bereich von einigen Minuten, beispielsweise 1 bis 15 Minuten.

Nach der Bestrahlung kann noch eine Nachtemperung (Vergütung) angeschlossen werden. Diese Nachtemperung wird zweckmäßigerweise bei einer Temperatur oberhalb der Glastemperatur der Polymerkomponente (a) durchgeführt. Für Polymethylmethacrylat enthaltende Formkörper ist eine Temperatur von 120 bis 150°C und eine Temperzeit von 3 bis 12 Stunden gut geeignet. Durch diese Temperung werden eventuell vorhandene Spannungen abgebaut und der erfindungsgemäß niedrige Restmonomergehalt noch weiter herabgesetzt.

Die nach dem erfindungsgemäßen Verfahren hergestellten Formkörper weisen eine besonders hohe mechanische Festigkeit und Lösungsmittelbeständigkeit auf. Besonders hervorzuheben ist die gegenüber Materialien des Standes der Technik deutlich verbesserte Verschleißfestigkeit.

### Beispiel 1

### Herstellung eines Granulates

In einem Compoundierschneckenextruder mit nachgeschaltetem Granulator werden die nachfolgend aufgeführten Komponenten homogen vermischt und zu einem Granulat mit einem Durchmesser von ca. 5 mm verarbeitet:
- 2.585,60 g: Polymethylmethacrylat-Perpolymerisat (Molekulargewicht Mw 450.000)
- 976,00 g: Bis-GMA 2,2-Bis-[p-(2'-hydroxy-3'-methacryloxypropyl)-phenyl]-propan
- 416,00 g: Triethylenglykoldimethacrylat
- 5,60 g: Campherchinon
- 14,00 g: p-Dimethylamino-benzolsulfonsäure-N,N-diallylamid
- 2,80 g: 2,6-Di-tert.-butylkresol.

Die Temperatur der Masse im Extruder betrug 140°C, die Viskosität der Schmelze betrug bei dieser Temperatur 2.000 Pa.s, gemessen bei einer Scherrate von 100/s. Das erhaltene Granulat wurde bei Herstellung, Lagerung und Weiterverarbeitung Vor Lichteinwirkung geschützt.

### Beispiel 2

### Spritzguß

Mit einer Spritzgießmaschine des Typs Engel 433 wurde das Granulat aus Beispiel 1 in Metallformen zu Zähnen verspritzt. Dabei wurden die folgenden Versuchsbedingungen eingehalten:

| | |
|---|---|
| Temperatur der Schmelze: | 140 bis 150°C |
| Temperatur der Düse: | 160°C |
| Temperatur des Werkzeuges: | 35 bis 40°C |
| Schließkraft: | 600 kN |
| Schneckendrehzahl: | 200/min |
| Einspritzzeit: | 4 s |
| Nachdruckzeit: | 12s |
| Kühlzeit: | 20s |
| Pausenzeit: | 3 s |
| Zycluszeit: | ca. 40s. |

### Beispiel 3

### Untersuchung der Photopolymerisation

Die Photopolymerisation des Gemisches aus Beispiel 1 wurde in einer Photo-DSC untersucht, indem bei 140°C hergestellte zylindrische Probekörper (Durchmesser: 5 mm, Höhe: 1 mm) im DSC-Gerät mit einer 75 W Halogenlampe mit Wärmeschutzfilter bestrahlt wurden. Bei 50, 70 und 90°C wurde sowohl die Reaktionsentalpie (Delta-H, als Maß für den Polymerisationsumsatz) als auch die Zeit bis zum Erreichen der maximalen Reaktionsgeschwindigkeit (t-max) gemessen:

| Meßtemperatur | Delta-H | t-max |
|---|---|---|
| 50°C | 21 J/g | 5,6 min |
| 70°C | 36 J/g | 2,9 min |
| 90°C | 51 J/g | 2,0 min |

Die Reaktionsenthalpie von 51 J/g entspricht einem nahezu vollständigen Umsatz, was durch infrarotspektroskopische Bestimmung des Doppelbindungsgehaltes bestätigt wurde.

### Beispiel 4

### Photopolymerisation

Spritzgegossene Zähne aus Beispiel 2 wurden in einem mit Halogenlampen ausgerüsteten Lichtofen (Leistung 75 W) unter Stickstoffspülung 8 Minuten bei 50°C und weitere 5 Minuten bei 90°C bestrahlt und anschließend 3 Stunden bei 130°C getempert. Die erhaltenen Kunststoffzähne wiesen eine hohe Härte, hohe Verschleißfestigkeit und gute Lösungsmittelbeständigkeit auf.

## Patentansprüche (Patentansprüche für folgende(n) Vertragsstaat(en): AT, CH, DE, ES, FR, GB, IT, LI, NL, SE)

1. Verfahren zur Herstellung von Dentalformkörpern, dadurch gekennzeichnet, daß härtbare, thermoplastisch verarbeitbare Zusammensetzungen enthaltend
a) 40 bis 90 Gew.-% Polymerkomponente mit einem Löslichkeitsparameter von 8-12,5 (cal/cm³)^{1/2} aus 40-100 Gew.-% unvernetztem und 0-60 Gew.-% vernetztem Polymer
b) 10 bis 60 Gew.-% polyfunktionelle (Meth)acrylsäureester
und
c) 0,1 bis 5 Gew-% Photoaktivator
thermoplastisch zu Formkörpern verarbeitet und anschließend durch Photopolymerisation vernetzt werden.

2. Verfahren gemäß Anspruch 1, dadurch gekennzeichnet, daß die härtbare, thermoplastisch verarbeitbare Zusammensetzung
a) 40 bis 90 Gew.-% Polymerkomponente mit einem Löslichkeitsparameter von 8-12,5 (cal/cm³)^{1/2} aus unvernetztem Polymer
b) 10 bis 60 Gew.-% polyfunktionelle (Meth)acrylsäureester
und
c) 0,1 bis 5 Gew.-% Photoaktivator
enthält.

3. Dentalformkörper, hergestellt gemäß Ansprüchen 1 und 2.

4. Dentalformkörper gemäß Anspruch 3, dadurch gekennzeichnet, daß es sich bei diesen um künstliche Zähne handelt.

## Patentansprüche (Patentansprüche für folgende(n) Vertragsstaat(en): BE, DK, GR, LU, PT)

1. Härtbare thermoplastisch verarbeitbare Zusammensetzung zur Herstellung von Dentalformkörpern, dadurch gekennzeichnet, daß die Zusammensetzung
a) 40 bis 90 Gew.-% Polymerkomponente mit einem Löslichkeitsparameter von 8-12,5 (cal/cm³)^{1/2} aus 40-100 Gew.-% unvernetztem und 0-60 Gew.-% vernetztem Polymer
b) 10 bis 60 Gew.-% polyfunktionelle (Meth)acrylsäureester
und
c) 0,1 bis 5 Gew-% Photoaktivator
enthält.

2. Härtbare Zusammensetzung nach Anspruch 1 dadurch gekennzeichnet, daß die Zusammensetzung
a) 40 bis 90 Gew.-% Polymerkomponente mit einem Löslichkeitsparameter von 8-12,5 (cal/cm³)^{1/2} aus unvernetztem Polymer
b) 10 bis 60 Gew.-% polyfunktionelle (Meth)acrylsäureester
und
c) 0,1 bis 5 Gew.-% Photoaktivator
enthält.

3. Dentalformkörper dadurch gekennzeichnet, daß sie eine gehärtete Zusammensetzung gemäß Ansprüchen 1 und 2 enthalten.

4. Dentalformkörper gemäß Anspruch 3, dadurch gekennzeichnet, daß es sich bei diesen um künstliche Zähne handelt.

5. Verfahren zur Herstellung von Dentalformkörpern, dadurch gekennzeichnet, daß die härtbaren Zusammensetzungen gemäß Ansprüchen 1 und 2 thermoplastisch zu Formkörpem verarbeitet und anschließend durch Photopolymerisation vernetzt werden.

## Claims (Claims for the following Contracting State(s): AT, CH, DE, ES, FR, GB, IT, LI, NL, SE)

1. Process for the production of dental mouldings, characterized in that curable compositions processible by a thermoplastic method and containing
a) 40 to 90% by weight of a polymer component having a solubility parameter of 8-12.5 (cal/cm³)^{½} and comprising 40-100% by weight of uncrosslinked and 0-60% by weight of crosslinked polymer,
b) 10 to 60% by weight of polyfunctional (meth)acrylates
and
c) 0.1 to 5% by weight of photoactivator
are processed by a thermoplastic method to give mouldings and are then crosslinked by photopolymerization.

2. Process according to Claim 1, characterized in that the curable composition processible by a thermoplastic method contains
a) 40 to 90% by weight of a polymer component having a solubility parameter of 8-12.5 (cal/cm³)^{½} and comprising uncrosslinked polymer,
b) 10 to 60% by weight of polyfunctional (meth)acrylates
and
c) 0.1 to 5% by weight of photoactivator.

3. Dental mouldings produced according to Claims 1 and 2.

4. Dental mouldings according to Claim 3, characterized in that they are artificial teeth.

## Claims (Claims for the following Contracting State(s): BE, DK, GR, LU, PT)

1. Curable composition, processible by a thermoplastic method, for the production of dental mouldings, characterized in that the composition contains
a) 40 to 90% by weight of a polymer component having a solubility parameter of 8-12.5 (cal/cm³)^{½} and comprising 40-100% by weight of uncrosslinked and 0-60% by weight of crosslinked polymer,
b) 10 to 60% by weight of polyfunctional (meth)acrylates
and
c) 0.1 to 5% by weight of photoactivator.

2. Curable composition according to Claim 1, characterized in that the composition contains
a) 40 to 90% by weight of a polymer component having a solubility parameter of 8-12.5 (cal/cm³)^{½} and comprising uncrosslinked polymer,
b) 10 to 60% by weight of polyfunctional (meth)acrylates
and
c) 0.1 to 5% by weight of photoactivator.

3. Dental mouldings characterized in that they contain a cured composition according to Claims 1 and 2.

4. Dental mouldings according to Claim 3, characterized in that they are artificial teeth.

5. Process for the production of dental mouldings, characterized in that the curable compositions according to Claims 1 and 2 are processed by a thermoplastic method to give mouldings and are then crosslinked by photopolymerization.

## Revendications (Revendications pour l'(les) Etat(s) contractant(s) suivant(s): AT, CH, DE, ES, FR, GB, IT, LI, NL, SE)

1. Procédé de production d'objets dentaires moulés, procédé caractérisé en ce qu'on soumet à transformation thermoplastique, pour obtenir des objets moulés, des compositions transformables par voie thermoplastique, durcissables et contenant :
a) 40 à 90 % en poids d'un composant polymère ayant un paramètre de solubilité de 8 à 12,5 (cal/cm³)^{1/2}, composant formé de 40 à 100 % en poids de polymère non réticulé et de 0 à 60 % en poids de polymère réticulé,
b) 10 à 60 % en poids d'un ester d'acide (méth)acrylique polyfonctionnel,
et
c) 0,1 à 5 % en poids d'un photoactivateur,
et en ce qu'on provoque ensuite la réticulation par photopolymérisation.

2. Procédé selon la revendication 1, caractérisé en ce que la composition durcissable, transformable par voie thermoplastique, contient
a) 40 à 90 % en poids d'un composant polymère ayant un paramètre de solubilité de 8 à 12,5 (cal/cm³)^{1/2}, composant formé d'un polymère non réticulé,
b) 10 à 60 % en poids d'un ester d'acide (méth)acrylique polyfonctionnel,
et
c) 0,1 à 5 % en poids d'un photoactivateur.

3. Objets dentaires conformés ou moulés, produits selon les revendications 1 et 2.

4. Objets dentaires moulés ou conformés selon la revendication 3, caractérisés en ce qu'il s'agit dans ce cas de dents artificielles.

## Revendications (Revendications pour l'(les) Etat(s) contractant(s) suivant(s): BE, DK, GR, LU, PT)

1. Composition durcissable transformable par voie thermoplastique pour la production d'objets dentaires conformés ou moulés, composition caractérisée en ce qu'elle contient :
a) 40 à 90 % en poids d'un composant polymère ayant un paramètre de solubilité de 8 à 12,5 (cal/cm³)^{1/2}, composant formé de 40 à 100 % en poids d'un polymère non réticulé et de 0 à 60 % en poids d'un polymère réticulé,
b) 10 à 60 % en poids d'un ester d'acide (méth)acrylique polyfonctionnel,
et
c) 0,1 à 5 % en poids d'un photoactivateur.

2. Composition durcissable selon la revendication 1, caractérisée en ce que la composition contient
a) 40 à 90 % en poids d'un composant polymère ayant un paramètre de solubilité de 8 à 12,5 (cal/cm³)^{1/2}, composant formé d'un polymère non réticulé,
b) 10 à 60 % en poids d'un ester d'acide (méth)acrylique polyfonctionnel,
et
c) 0,1 à 5 % en poids d'un photoactivateur.

3. Objets dentaires conformés ou moulés, caractérisés en ce qu'ils contiennent une composition durcie, selon les revendications 1 et 2.

4. Objets dentaires moulés ou conformés selon la revendication 3, caractérisés en ce qu'il s'agit dans ce cas de dents artificielles.

5. Procédé pour produire des objets dentaires moulés ou conformés, caractérisé en ce qu'on traite des compositions durcissables, selon les revendications 1 et 2, pour les transformer par voie thermoplastique en des objets moulés ou conformés et en ce qu'on les soumet ensuite à réticulation par photopolymérisation.
